# NEUE EUROPÄISCHE PATENTSCHRIFT

(11) **EP 1 773 215 B2**
(45) Veröffentlichungstag und Bekanntmachung des Hinweises auf die Entscheidung über den Einspruch: **24.07.2019**
(45) Hinweis auf die Patenterteilung: 15.12.2010
(21) Anmeldenummer: 05772204.3
(22) Anmeldetag: 20.07.2005
(51) Int. Cl.: A61B 17/32

(54) **CHIRURGISCHES SCHNEIDINSTRUMENT**
SURGICAL CUTTING INSTRUMENT
INSTRUMENT DE COUPE CHIRURGICAL

(30) Priorität: 22.07.2004 CH 12432004
(43) Veröffentlichungstag der Anmeldung: 18.04.2007
(73) Patentinhaber: Da Rold Engineering AG, 4500 Solothurn (CH)
(72) Erfinder: SADRI, Hassan, CH-1632 Riaz (CH)
(74) Vertreter: Fleck, Hermann-Josef
(86) Internationale Anmeldenummer: PCT/IB2005/052425
(87) Internationale Veröffentlichungsnummer: WO 2006/011119

(56) Entgegenhaltungen:
- EP-A- 0 499 465
- US-A- 4 811 734
- US-A- 5 601 583
- US-A- 5 643 303
- US-A- 5 709 698
- US-A- 5 766 199
- US-A- 5 843 106
- US-A- 6 053 928
- US-B1- 6 217 598
- US-B1- 6 419 684
- US-B1- 6 428 539
- US-B1- 6 620 180

## Beschreibung

Die vorliegende Erfindung betrifft ein chirurgisches Schneidinstrument gemäss Oberbegriff des Patentanspruchs 1.

Chirurgische Schneidinstrumente für die Anwendung bei geschlossener oder endoskopischer Chirurgie sind z.B. aus EP 0 557 044 oder US-B-6 217 598 bekannt, wobei das Dokument US-B-6 217 598 die im Oberbegriff des Anspruchs 1 definierten Merkmale offenbart. Sie bestehen aus zwei ineinander geschobenen Rohren, an denen sich am einen Ende im Endbereich der sogenannte "Schneidkopf" und am andern Ende eine Kupplung für die Verbindung mit der Antriebsturbine befindet. Die Endstücke sind nach vorne offen oder abgeschlossen und weisen ebenfalls nach vorne gerichtet seitliche Öffnungen auf, durch die Spülflüssigkeit und während des Eingriffes abgetrenntes Gewebe abgeführt wird.

Die Rohre solcher Instrumente sind von kleinem Durchmesser und grosser Länge, um ohne grosse Operationswunden zu verursachen, Eingriffe im Körper zu ermöglichen. Man arbeitet normalerweise mittels drei im Gewebe angelegten Öffnungen, jedoch im gegen Aussen geschlossenen Bereich des Gewebes. Durch die erste Öffnung wird eine Kamera zur Beobachtung der Arbeitsstelle über den Bildschirm in den Körper eingebracht, die zweite Öffnung dient dem Zugang für die chirurgischen Schneidinstrumente, und durch eine dritte Öffnung wird eine Düse eingebracht. Durch diese Düse bringt man Spül,flüssigkeit zum Arbeitsfeld am Ort des eigentlichen Eingriffes. Diese Spülflüssigkeit wird gemeinsam mit dem abgetrennten Gewebe durch das rohrförmige Zentrum des Schneidwerkzeuges, wieder aus dem Körper entfernt.

Um das Arbeitsfeld zu vergrössern wird z.B. bei Eingriffen im Hüftgelenk Femur und Becken durch einen invasiven Distraktor oder durch einfache Dehnung in dem Rahmen, in dem Sehnen, Bänder, Nerven und Muskeln dies zulassen auseinander gespreizt. Die Kamera dient der Überwachung des Arbeitsfeldes über Fernsehbildschirme während der Operation.

Mit der Spülflüssigkeit wird einesteils das Arbeitsfeld sauber gehalten und anderseits werden die mit dem Schneidinstrument abgetrennten Teile mit der Spülflüssigkeit vom Arbeitsfeld abgesogen. Es gibt auf dem Markt auch Instrumente mit Kamera, durch die gleichzeitig die Spülflüssigkeit zum Arbeitsfeld gebracht wird. Die kombinierte Vorrichtung Kamera mit Flüssigkeitszuführung ermöglicht es, mit nur zwei Eingriffstellen zu arbeiten. In manchen Fällen ist die Kombination von Kamera mit Flüssigkeitszuführung aus praktischen Gründen nicht möglich, z.B. weil man die Kamera aus operationstechnischen Gründen an anderer Stelle anbringen will um einen ganz bestimmten Einblick auf das Arbeitsfeld zu haben.

Die der Erfindung zugrunde liegenden chirurgischen Schneidinstrumente sind wie oben beschrieben als lange Rohre ausgebildet. Durch das Innere, sich drehende Rohr, wird die Spülflüssigkeit zusammen mit den abgetrennten Teilen abgesogen. Wichtige zu lösende Probleme für die Herstellung solcher chirurgischer Schneidinstrumente sind Herstellungstechnik, Materialwahl und Wirtschaftlichkeit. Geeignetes, sterilisierbares Material muss in der Art bearbeitet werden, dass bei wirtschaftlicher Herstellung ein möglichst präzises Schneidinstrument entsteht. Normalerweise werden solche Instrumente aus hochwertigen metallischen Legierungen gefertigt.

Um mit dem chirurgischen Schneidinstrument möglichst effizient arbeiten zu können, sollte es scharfe Schneidkanten aufweisen. Die Präzision mit der die Schneiden des Innenrohres und die Schneiden des Aussenrohres einander nähern ist, zusammen mit den scharfen Kanten der Schneiden, entscheidend dafür, wie leicht sich Gewebe abtrennen lässt. Die am schwierigsten zu erfüllende Anforderung an das chirurgische Schneidinstrument ergibt sich daraus, dass in demselben Eingriff verschiedenartige Gewebestrukturen geschnitten und entfernt werden müssen. Nun ist hinlänglich bekannt, dass ein Schneidwerkzeug für sprödes und hartes Material nicht einfach und selbstverständlich für das Schneiden von elastischen, faserigen oder plastisch weichen Materialien geeignet ist. Dies ist aber gerade das, was sich der Chirurg wünscht, um den Eingriff in kurzer Zeit und effizient vornehmen zu können.

Dieser Forderung kommt z.B. EP 0 557 044 in der Art nach, dass man die Schneidkanten des Innenrohres mit Sägezahnartigen Schneiden ausrüstet. Man erreicht dadurch, dass elastisches und hartes Gewebe "gepackt" werden kann. Hierin besteht aber auch das mit dieser Art der Schneidkantenausbildung entstehende Problem. Das Werkzeug packt sich Gewebe und hat die Tendenz sich im Gewebe zu verhaken. Verhakt sich das Schneidinstrument, steht es entweder durch das Ansprechen einer Überlastkupplung still, oder es reisst aus dem zu behandelnden Gewebe zu grosse Teile heraus.

Herkömmliche chirurgische Schneidwerkzeuge sind am vorderen Ende abgerundet. Dies begründet sich im Gedanken, dass ein abgerundetes Instrument leichter in das Gewebe eingebracht werden kann. Es hat seinen Ursprung aber auch in der einfacheren Herstellungstechnik, wenn an einem langen Rohr ein Abschluss angebracht werden muss. Man kann auf Methoden wie Tiefziehen oder Bördeln etc. zurückgreifen. Alle diese auf dem Markt vorhandenen chirurgischen Schneidwerkzeuge sind mechanisch und von den Toleranzen her ungenau. Die Herstellungstechniken lassen nichts anderes zu. Zudem muss für Material das tief gezogen werden kann weiches Material sein, es darf schon gar nicht spröde oder hart sein. Es sind dem Erfinder keine Werkzeuge dieser Art bekannt, die gehärtet wären.

In der Operation hat man die Erfahrung, dass dies folgende nachteilige Effekte haben kann, die im folgenden aufgezählt sind:
- Das weiche Material des Aussenrohres und des
- Innenrohres können "anfressen".
- Das Material muss für die chirurgischen Schneidwerkzeuge vernickelt werden, was die Gefahr des Abbröckelns dieser Schicht mit sich bringt.
- Durch die Methoden Tiefziehen oder Bördeln erreicht man niemals eine schöne definierte Oberfläche und kann auch keine Rundung genau bestimmen.
- Unrunde, harte Innenrohre, die sich in Unrunden aber in, aus weichem Material gefertigten, Aussenrohren drehen verursachen Metallabrieb der sich im Gewebe festsetzen und Beschwerden hervorrufen kann.

Die vorliegende Erfindung stellt sich nunmehr die Aufgabe ein Chirurgisches Schneidinstrument der eingangs genannten Art derart zu verbessern, dass es die harten Bedingungen für die in Vivo Anwendung ohne Probleme erfüllt und dem Chirurgen ein wirklich scharfes Schneidinstrument für alle Arten des Gewebes und der Knochen dienen kann.

Diese Aufgabe löst ein Chirurgisches Schneidinstrument mit den Merkmalen des Patentanspruches 1. Weitere erfindungsgemässe Merkmale gehen aus den abhängigen Ansprüchen hervor und deren Vorteile sind in der nachfolgenden Beschreibung erläutert.

In der Zeichnung zeigt:
- Fig 1: Ansicht des Aussenrohres mit Kupplungselement
- Fig 2: Ansicht des Innenrohres mit Kupplungselement
- Fig 3: Längsschnitt,durch das Aussenrohr
- Fig 4: Längsschnitt durch das Innenrohr
- Fig 5: Längsschnitt durch zusammengestelltes Aussen- und Innenrohr
- Fig 6: Querschnitt an der Stelle A - A durch zusammengestellte Aussen- und Innenrohre
- Fig 7: Querschnitt an der Stelle A - A durch zusammengestellte Aussen- und Innenrohre
- Fig 8: Längsschnitt durch das Aussenkopfstück
- Fig 9: Querschnitt an der Stelle B - B durch ein Aussenkopfstück
- Fig 10: Längsschnitt durch das Innenkopfstück
- Fig 11: Querschnitt an der Stelle C - C durch ein Innenkopfstück mit einer Innenöffnung
- Fig 12: Ansicht eines Innenkopfstückes mit einer oder zwei Innenöffnungen
- Fig 13: Ansicht eines Innenkopfstück mit einer oder zwei Innenöffnungen um 90° gedreht
- Fig 14: Querschnitt an der Stelle D - D durch ein Innenkopfstück mit zwei Innenöffnungen
- Fig 15: Querschnitt an der Stelle D - D durch ein Innenkopfstück mit drei Innenöffnungen
- Fig 16: Dreidimensionale Ansicht eines Innenkopfstückes mit drei Innenöffnungen.
- Fig 17: Andsicht eines Innenkopfstückes mit einer oder zwei Innenöffnungen und einem Fräser an der Spitze

Die Figuren stellen bevorzugte Ausführungsbeispiele dar, welche mit der nachfolgenden Beschreibung erläutert werden.

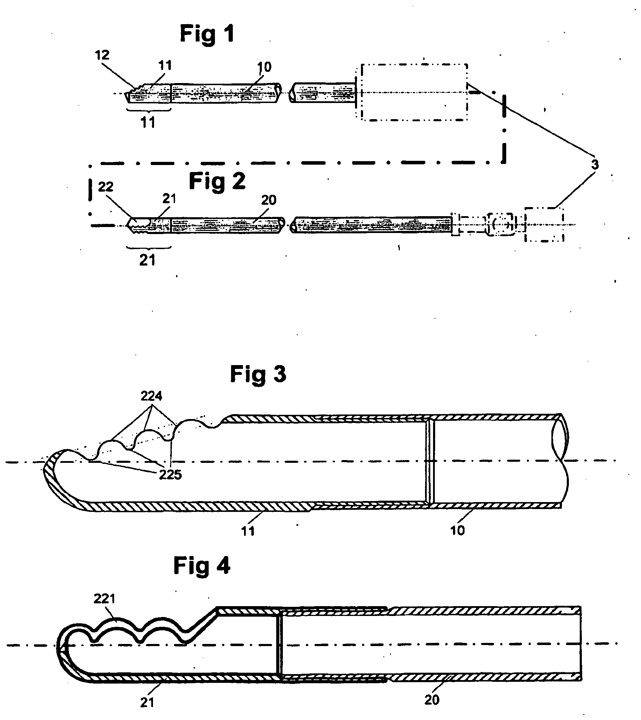

Ein Chirurgisches Schneidinstrument nach dem Stand der Technik dieser Erfindung besteht aus einem festen Aussenrohr 10 (Fig 1) und einem sich darin drehenden Innenrohr 20 (Fig 2). Das ganze Schneidinstrument wird an einem dafür vorgesehenen Antrieb angeschlossen, so dass das sich das Innenrohr 20 meist mit einem Turbinenantrieb drehen lässt und gleichzeitig durch die Mitte des rohrförmigen Instrumentes mittels Vakuum eine Absaugung gewährleistet ist. Solche Vorrichtungen sind in der arthroskopischen Behandlung mittels kleinen chirurgischen Eingriffen überall im Einsatz.

Bei bekannten Konstruktionen sind Aussenrohr 10 und Aussenkopfstück 11 aus einem Stück gefertigt. Dies gilt ebenso für das Innenrohr 20 und das Innenkopfstück 21. Da in der klinischen Anwendung solche Instrumente ohne Tadel sauber und steril sein müssen, werden für die Herstellung spezielle Legierungen verwendet. Die anspruchsvolle Technik zur Schweissung solcher Materialien und Materialstärken ist der Grund,dafür, dass die Rohre üblicherweise in einem Stück hergestellt werden. Der Erfinder hat nun Methoden entwickelt, welche eine sichere Schweissung zwischen Aussenrohr 10 und Aussenkopfstück 11 (Fig 3), sowie zwischen Innenrohr 20 und Innenkopfstück 21 (Fig 4) ermöglichen. Dadurch werden andere Bearbeitungsmethoden für die Kopfstücke möglich.

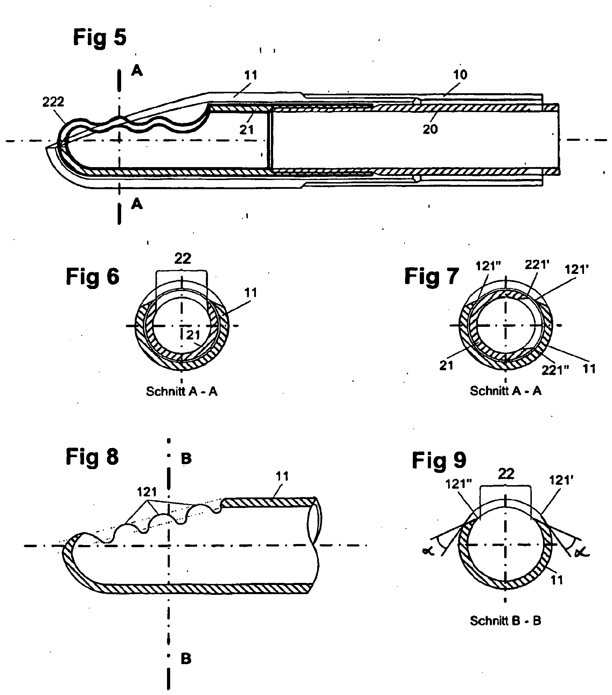

Die erfindungsgemässe Schneidvorrichtung unterscheidet sich wesentlich von den marktüblichen Produkten. Die Aussenschneidkanten 121' und 121" des Aussenkopfstückes 11 bilden zur Tangente nach innen einen spitzen Winkel α. Die Aussenschneidkanten 121' und 121" befinden sich also unmittelbar am Innendurchmesser des Aussenkopfstückes 11. Damit nur eine kleine Schnittkraft bei guten Schnitteigenschaften erreicht wird, bilden die Innenschneidkanten 221' und 221" zur Tangente nach aussen einen spitzen Winkel β, so dass sich die Innenschneidkanten 221' und 221" am Aussendurchmesser des Innenkopfstückes 21 befinden. Dreht sich nun das Innenkopfstück 21 im Uhrzeigersinn (Fig 7), so bewegt sich die Innenschneidkante 221' sehr nahe an der Aussenschneidkante 121' vorbei. Die gewählte Geometrie der Form einer Verzahnung über die Länge der Schnittkanten 121, 221 ergibt den Effekt, dass die Schneidkanten 121 und 221 während des Schnittvorganges die Tendenz haben zusammen zu rücken. Dies ergibt nochmals eine verbesserte Schnitteigenschaft, weil damit zwischen den Schneidkanten 121 und 221 praktisch kein Spiel mehr vorhanden ist. Da beide Schneidkanten, sowohl die Aussenschneidkante 121' als auch die Innenschneidkante 221' einen kleinen Schnittwinkel aufweisen, wird jede Art Gewebe oder Knochen, ob weich, elastisch, hart oder faserig, sauber abgetrennt. Kein Gewebe wird abgequetscht oder gar abgerissen. Dasselbe geschieht in gleicher Form bei umgekehrter Drehrichtung zwischen Innenschneidkante 221" und Aussenschneidkante 121".

Das Schneidinstrument kann in beiden Drehrichtungen und oszillierend mit exakt derselben Schneideigenschaft und -qualität eingesetzt werden. Dadurch können Schneidinstrumente mit kleinerem Durchmesser verwendet werden. Dies ist einzigartig und bietet vor allem bei engen operativen Verhältnissen grosse Vorteile. Als Beispiel sei hier der Eingriff für eine Miniskus-Operation erwähnt. Für Eingriffe in Gelenken kann dadurch das Mass der Distraktion kleiner gehalten werden, was das Risiko einer Überdehnung von Sehnen, Muskeln und Nerven reduziert.

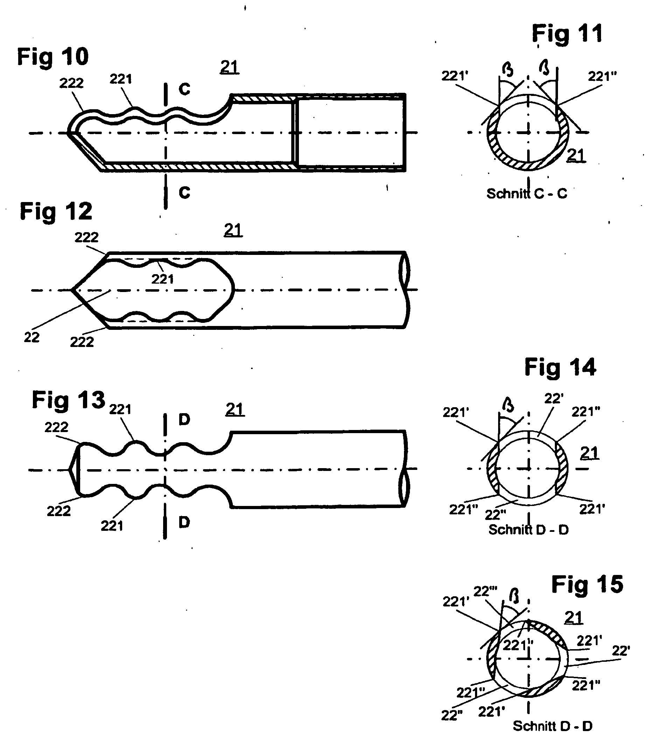

Die Form dieses Schneidinstrumentes ermöglicht in vielen Bereichen den Einsatz auch dort, wo traditioneller weise Fräser zum Einsatz kommen würden. Die Aussenschneidkanten 121 (Fig3) und die Innenschneidkanten 221 (Fig10) sind über ihre Länge wellenförmig ausgebildet. Dadurch gibt es praktisch keine Momente, zu denen sich die ganze Schneide gleichzeitig im Einsatz befindet. Dies erlaubt mit kleinen Antriebskräften effizient und schnell arbeiten zu können. Die in Fig. 3 dargestellte Wellenform der Zähne, breiter Zahn 224 und schmale Zahnlücke 225 ist für dieses chirurgische Schneidinstrument typisch.

Wie oben beschrieben sind herkömmliche Schneidwerkzeuge am vorderen Ende abgerundet. Wie oben erwähnt, können beim vorgestellten chirurgischen Schneidinstrument Aussenkopfstück 11 und Innenkopfstück 21 nach deren Vorfertigung mit dem Aussenrohr 10 respektive dem Innenrohr 20 verbunden werden. Das ermöglicht andere Formen und die Anwendung anderer Fertigungstechniken zur Ausbildung der beiden Kopfstücke 11, 21.

Der Erfinder schlägt vor allem einen kegeligen Abschluss des Chirurgischen Schneidmessers vor. Wie in Fig 5 dargestellt entsteht bei dieser Ausbildung des Aussenkopfstückes 11 und des Innenkopfstückes 21 eine Schneidecke 222 die zur Schneidkante 121 des Aussenkopfstückes 11 leicht vorsteht. Mit dieser Schneidecke 222 kann der Chirurg sehr gezielt schneiden. Eine gängige Methode ist beispielsweise, dass er mittels seitlicher Schneidecke 222 eine grubenartige Vertiefung im abzutragenden Flächengewebe einschneidet. Im Monitor kann er auf diese Weise erkennen, wie viel Material er von der Fläche um die Furche herum bereits abgetragen hat. Dies ist eine grosse Hilfe als Referenzlinie zur Abschätzung des Materialabtrages während der Operation. Im Monitor können die relativen Grössenordnungen nur mit grosser Erfahrung und nur unpräzise erkannt werden. Diese Form des Chirurgischen Schneidinstrumentes ermöglicht deshalb schnelleres Arbeiten, ohne dass sich daraus ein grösseres Risiko ergibt.

Die grösste Neuerung, welche erst aufgrund neuer Herstellungstechniken und Wahl der richtigen Materialien möglich wurde, ist das Ausbilden des Innenkopfstückes mit ein, zwei oder drei Innenöffnungen 22 (Fig. 12). Das zu lösende Problem war die Festigkeit des Materials des Innenkopfstückes. Dieses muss sehr zäh und trotzdem schweissbar sein. Ein Material das beide Eigenschaften aufweist wurde gefunden. Der grosse Vorteil den man mit zwei (Fig 13,14) oder gar drei (Fig 15) Innenöffnungen 22 hat, ergibt sich beim Absaugen von Spülflüssigkeit und Gewebe. Je besser die Schnittleistung und je grösser die Öffnung durch die man das abgetrennte Material abführen kann, desto sauberer ist das Arbeitsfeld und desto schneller und genauer kann der Chirurg arbeiten.

Das Aussenrohrkopfstück 11 bleibt bei,allen Formen der Innenrohrkopfstücke 21 immer gleich. Jede Innenöffnung, ob eine, zwei oder drei vorhanden sind, hat jeweils zwei Innenschneidkanten 221',221". Diese sind auch immer gleich ausgebildet, so dass mit dem Schneidinstrument mit einer Innenöffnung (Fig 10,11,12), mit zwei Innenöffnungen (Fig 13,14) oder mit drei Innenöffnungen (Fig 15) in beiden Drehrichtungen gearbeitet werden kann.

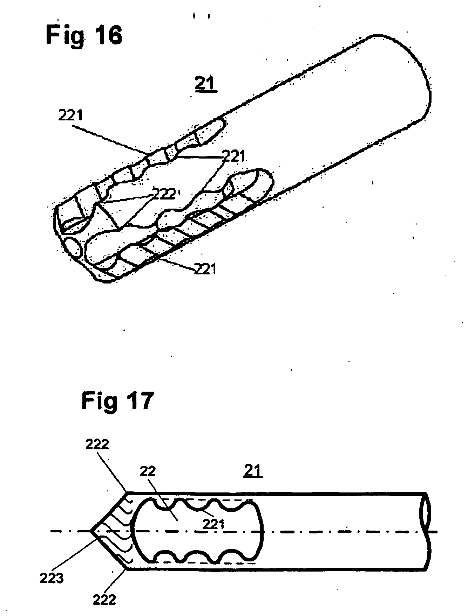

Im praktischen Einsatz gibt sich die Notwendigkeit auch einen Fräskopf 223 (Fig. 17) einzusetzen. Um die Vorteile des oben beschriebenen chirurgischen Schneidwerkzeuges 1 zu nutzen und trotzdem ein Fräswerkzeug zur Verfügung zu haben, wurde an einem chirurgischen Schneidwerkzeug 1 der kegelige Abschluss des Innenkopfstückes 21 als Fräskopf 223 ausgebildet. Diese Anordnung hat zum Vorteil, dass das abgefräste Material sofort zur Innenöffnung 22 gelangt und dort abgesogen werden kann. Dieser Fräskopf 223 ist als "Kappe" mit mehreren Fräszähnen ausgebildet. Man erstrebt eine möglichst hohe Zahl an Fräszähnen.

Die Ausbildung des Fräskopfes 223 als Kappe zusammen mit der Tatsache, dass das vorgestellte chirurgische Schneidwerkzeug mit extrem kleinen Spielen arbeitet, hat den Vorteil, dass keine Weichteile um den Fräskopf 223 gewickelt werden. Dies ist eines der grossen Probleme die herkömmliche Fräswerkzeuge, vor allem wenn das Aussenkopfstück 11 so ausgebildet ist, dass der Fräskopf 223 nach vorne absolut frei ist, in diesem Einsatz oft haben. Der Aussenrohrkopfstück 11 bleibt auch für den Einsatz dieses mit Fräskopf 223 ausgestatteten Innenkopfstück 21 wie in Fig 8 und Fig 9 dargestellt, immer gleich.

Versuche zeigten eindrücklich, dass bei gleicher Drehzahl des angetriebenen Innenrohres 20 eine weit grössere Schnittleistung erreicht werden kann. Ebenso wurde festgestellt, dass mit den erfindungsgemäss ausgestatteten Werkzeugen, insbesondere auch mit dem Innenkopfstück 21 mit Fräskopf 223 Fig 17 sehr viel weniger Kraft aufgewendet werden muss, als mit herkömmlichen Werkzeugen. Weitere durch den praktischen Einsatz erkennbare Vorteile sind:
- Das vorgestellte Schneidinstrument ermöglicht schnelleres Arbeiten und ist dadurch nicht nur aus wirtschaftlichen Gründen den herkömmlichen Werkzeugen weit überlegen.
- Das vorgestellte Schneidinstrument kann als Klingen-Fräser (ohne Fräskopf 223 auf dem Innenkopfstück 21) und als Fräser-Klinge (mit dem Fräskopf 223 auf dem Innenkopfstück 21) eingesetzt werden. Die Einsatzbereiche überlappen sich derart, dass mit beiden Werkzeugen Bereiche bearbeitet werden können, die mehrere bekannte Werkzeuge und damit Werkzeugwechsel während der Operation ersetzen.
- Durch die gewählte Form der Schneidengeometrie ist die Verletzungsgefahr während des Einbringens des Werkzeuges in das Gewebe reduziert.
- Die runden Zähne sind um ein vielfaches robuster, als die spitzen Zähne der bekannten Werkzeuge.
- Die kegelige Ausbildung des Innenkopfstückes 21 erlaubt es, dasselbe als Bohrer einzusetzen.
- Die von den Rohren (Innenrohr 20 resp. Aussenrohr 10) getrennte Fertigung der Kopfstücke (Innenkopfstück 21 resp. Aussenkopfstück 11) erlaubt die Einhaltung von kleinen und immer gleichen Toleranzen, unabhängig vom Durchmesser des Werkzeuges.

## Patentansprüche

1. Chirurgisches Schneidinstrument grosser Länge, bestehend aus einem Aussenrohr (10), welches im Endbereich (2) mit einem Aussenkopfstück (11) verbunden ist, das mindestens eine seitlich nach vorne gerichtete Aussenöffnung (12) mit mindestens zwei Aussenschneidkanten (121', 121") aufweist und einem Innenrohr (20), welches im Endbereich (2) mit einem Innenkopfstück (21) verbunden ist, das mindestens eine seitlich nach vorne gerichtete Innenöffnung (22) mit mindestens zwei über ihre Länge wellenförmig verlaufenden Innenschneidkanten (221', 221") aufweist, wobei das Innenrohr (20) im Aussenrohr (10) drehbar gelagert ist, und sich bei relativer Drehung des Innenrohres (20) im Aussenrohr (10) die Aussenschneidkanten (121', 121") und die Innenschneidkanten (221', 221") mit wenig Spiel aneinander vorbei bewegen, **dadurch gekennzeichnet, dass** die Aussenschneidkanten (121', 121") zur Tangente nach innen einen, spitzen Winkel α von 10 - 90° bilden und über die Länge wellenförmig mit aufstehenden runden Zähnen (224) und runden Zahnlücken (225) verlaufen und die Innenschneidkanten (221', 221") zur Tangente nach aussen einen spitzen Winkel β von 10 bis 90° bilden und ebenfalls wellenförmig mit aufstehenden runden Zähnen und runden Zahnlücken verlaufen.

2. Schneidinstrument nach Anspruch 1, **dadurch gekennzeichnet, dass** das Aussenkopfstück (11) und das Innenkopfstück (21) im Endbereich eine kugelige Form zum Abschluss bilden.

3. Schneidinstrument nach Anspruch 1, **dadurch gekennzeichnet, dass** das Aussenkopfstück (11) und das Innenkopfstück (21) im Endbereich eine kegelige Form zum Abschluss bilden.

4. Schneidinstrument nach den Ansprüchen 1 und 2, **dadurch gekennzeichnet, dass** das Innenkopfstück (21) zwei Innenöffnungen (22', 22") mit jeweils zwei Innenschneidkanten (221', 221") aufweist.

5. Schneidinstrument nach den Ansprüchen 1 und 3, **dadurch gekennzeichnet, dass** das Innenkopfstück (21) zwei Innenöffnungen (22', 22") mit jeweils zwei Innenschneidkanten (221', 221") aufweist.

6. Schneidinstrument nach Anspruch 1 und 2, **dadurch gekennzeichnet, dass** das Innenkopfstück (21) drei Innenöffnungen (22', 22", 22"') mit jeweils zwei Innenschneidkanten (221', 221") aufweist.

7. Schneidinstrument nach Anspruch 1 und 3, **dadurch gekennzeichnet, dass** das Innenkopfstück (21) drei Innenöffnungen (22', 22", 22"") mit jeweils zwei Innenschneidkanten (221', 221") aufweist.

8. Schneidinstrument nach Ansprüchen 1 bis 7, **dadurch gekennzeichnet, dass** das Innenkopfstück (21) am Ende einen Fräskopf (223) aufweist.

9. Schneidinstrument nach Anspruch 1, **dadurch gekennzeichnet, dass** die aufstehenden Zähne (224) mindesten doppelt so breit sind als die Zahnlücken (225).

## Claims

1. Surgical cutting instrument of great length, consisting of an outer tube (10) which, in the end area (2), is connected to an outer head part (11) which has at least one laterally and forwardly directed outer opening (12) with at least two outer cutting edges (121', 121"), and of an inner tube (20) which, in the end area (2), is connected to an inner head part (21) which has at least one laterally and forwardly directed inner opening (22) with at least two inner cutting edges (221', 221") extending along the length, the inner tube (20) being mounted rotatably in the outer tube (10), and, upon relative rotation of the inner tube (20) in the outer tube (10), the outer cutting edges (121', 121") and the inner cutting edges (221' , 221") move past one another with slight clearance, **characterized in that** the outer cutting edges (121', 121") form inwardly with respect to the tangent an acute angle α of 10-90° and extend along the length in an undulating configuration with round upright teeth (224) and round tooth gaps (225), and the inner cutting edges (221', 221") form outwardly with respect to the tangent an acute angle β of 10° to 90° and also extend in an undulating configuration with round upright teeth and round tooth gaps.

2. Cutting instrument according to Claim 1, **characterized in that** the outer head part (11) and the inner head part (21) in the end area form a spherical shape for closure.

3. Cutting instrument according to Claim 1, **characterized in that** the outer head part (11) and the inner head part (21) in the end area form a conical shape for closure.

4. Cutting instrument according to Claims 1 and 2, **characterized in that** the inner head part (21) has two inner openings (22', 22") with in each case two inner cutting edges (221' , 221").

5. Cutting instrument according to Claims 1 and 3, **characterized in that** the inner head part (21) has two inner openings (22', 22") with in each case two inner cutting edges (221' , 221").

6. Cutting instrument according to Claims 1 and 2, **characterized in that** the inner head part (21) has three inner openings (22', 22", 22"') with in each case two inner cutting edges (221', 221").

7. Cutting instrument according to Claims 1 and 3, **characterized in that** the inner head part (21) has three inner openings (22', 22", 22"') with in each case two inner cutting edges (221', 221").

8. Cutting instrument according to Claims 1 to 7, **characterized in that** the inner head part (21) has a milling head (223) at the end.

9. Cutting instrument according to Claim 1, **characterized in that** the upright teeth (224) are at least twice as wide as the tooth gaps (225).

## Revendications

1. Instrument de coupe chirurgical de grande longueur, composé d'un tube extérieur (10) relié dans la zone d'extrémité (2) à une partie de tête extérieure (11) comportant au moins une ouverture extérieure (12) orientée en côté vers l'avant avec au moins deux arêtes tranchantes extérieures (121', 121") et d'un tube intérieur (20) relié dans la zone d'extrémité (2) à une partie de tête intérieure (21) qui comporte au moins une ouverture intérieure (22) orientée en côté vers l'avant avec au moins deux arêtes tranchantes intérieures (221', 221") s'étendant sur la longueur, le tube intérieur (20) étant disposé de façon à pouvoir tourner dans le tube extérieur (10), les arêtes tranchantes extérieures (121' , 121") et les arêtes tranchantes intérieures (221', 221") avançant l'une contre l'autre, avec une rotation relative du tube intérieur (20) dans le tube extérieur (10) avec un faible jeu, **caractérisé en ce que** les arêtes tranchantes extérieures (121', 121") forment un angle aigu α de 10 - 90° vers l'intérieur par rapport à la tangente et s'étendent de manière ondulée sur la longueur, avec des dents saillantes (224) rondes et des interstices (225) ronds entre les dents, et **en ce que** les arêtes tranchantes intérieures (221', 221") forment un angle aigu β de 10 à 90° vers l'extérieur par rapport à la tangente, et s'étendent également de manière ondulée avec des dents saillantes rondes et des interstices ronds entre les dents.

2. Instrument de coupe selon la revendication 1, **caractérisé en ce que** la partie de tête extérieure (11) et la partie de tête intérieure (21) prennent une forme sphérique au niveau de leur terminaison, dans la zone d'extrémité.

3. Instrument de coupe selon la revendication 1, **caractérisé en ce que** la partie de tête extérieure (11) et la partie de tête intérieure (21) prennent une forme conique au niveau de leur terminaison, dans la zone d'extrémité.

4. Instrument de coupe selon les revendications 1 et 2, **caractérisé en ce que** la partie de tête intérieure (21) comporte deux ouvertures intérieures (22', 22") respectivement dotées de deux arêtes tranchantes intérieures (221', 221").

5. Instrument de coupe selon les revendications 1 et 3, **caractérisé en ce que** la partie de tête intérieure (21) comporte deux ouvertures intérieures (22' , 22") respectivement dotées de deux arêtes tranchantes intérieures (221', 221").

6. Instrument de coupe selon la revendication 1 et 2, **caractérisé en ce que** la partie de tête intérieure (21) comporte trois ouvertures intérieures (22', 22",22"') respectivement dotées de deux arêtes tranchantes intérieures (221', 221").

7. Instrument de coupe selon la revendication 1 et 3, **caractérisé en ce que** la partie de tête intérieure (21) comporte trois ouvertures intérieures (22', 22",22'") respectivement dotées de deux arêtes tranchantes intérieures (221', 221").

8. Instrument de coupe selon les revendications 1 à 7, **caractérisé en ce que** la partie de tête intérieure (21) comporte une tête de fraise (223) à son extrémité.

9. Instrument de coupe selon la revendication 1, **caractérisé en ce que** les dents (224) saillantes sont au moins deux fois plus larges que les interstices entre les dents (225).
